# EUROPEAN PATENT APPLICATION

(11) **EP 4 556 036 A1**
(43) Date of publication of application: **21.05.2025**
(21) Application number: 24179596.2
(22) Date of filing: 03.06.2024
(51) Int. Cl.: A61M 5/32

(54) **SAFETY MECHANISM FOR AN INJECTION DEVICE**

(71) Applicant: Ypsomed AG, 3401 Burgdorf (CH)
(72) Inventor: Klötzli, Urs, 3414 Oberburg (CH)
(74) Representative: Kleiner, Stefan

(57) **Abstract**

The invention relates to a safety mechanism comprising a cap (50) and a disposable reservoir unit (1) for an injection device (3) for dispensing a liquid drug through an injection needle (12). The reservoir unit (1) is configured for releasable attachment to a reusable drive unit (2) of the injection device. The reservoir unit (1) comprises a housing (40), a needle cover sleeve (20) movable between a covering position and a retracted position. The needle cover sleeve (20) further comprises a coupling portion (22) adapted to be releasably coupled to an actuation member (81) of the drive unit (2) to move the needle cover sleeve (20) between the covering position and the retracted position. In a shipping state of the injection device (3) the cap (50) is attached to a distal end of the reservoir unit (1). When the needle cover sleeve (20) is moved towards its covering position by the actuator the cap (50) is moved into a release position by the needle cover sleeve (20).

## Description

### FIELD OF THE INVENTION

The present invention relates to a disposable reservoir unit for an injection device for dispensing a liquid drug through an injection needle for injecting, delivering, administering, infusing or dispensing substances and/or liquids such as insulin, hormone preparations or vaccines. The reservoir comprises a housing, a needle cover sleeve movable along the longitudinal axis relative to the housing between a covering position and a retracted position. The needle cover sleeve comprises a coupling portion adapted to be releasably coupled to an actuator member of the drive unit to move the needle cover sleeve along the longitudinal axis.

### BACKGROUND OF THE INVENTION

A variety of diseases exist that require regular treatment by subcutaneous administration of a medicament, and several drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. Automatic injection devices may comprise an electronic dose dial mechanism to automatically set a dose.

Also known is the use of autoinjectors with prefilled syringes. Autoinjectors usually comprise a body for housing a syringe as well as a drive mechanism in order to move the plunger of the syringe upon actuation of the autoinjector. The drive mechanism typically comprises a source of drive, such as an electric motor or a mechanical strong spring for moving a transfer element, for example a rod, which acts on the plunger of the syringe. The prefilled syringe has a needle or cannula that is permanently attached to a first end of a syringe barrel or reservoir that is sealed at the opposing end by the plunger.

For safety and hygiene reasons it is desirable that the needle does not protrude from a housing of the autoinjector except for the time when the needle is used for injection of a medicament. Thus, either the autoinjector moves the needle out of the housing for the injection and back into the housing after injection or the autoinjector includes a needle guard or needle cover which may be moved to unsheathe the needle for injection and which may be moved back to a needle covering position after the injection.

The majority of autoinjectors are configured as single use devices which incorporate both the syringe and the drive mechanism in the same housing. Such devices are usually disposable for hygiene reasons.

Disposable autoinjectors comprising an electric actuator or an electronic control require a source of energy which is usually in the form of a battery. However, in this case, the autoinjectors should not be disposed in the regular waste but have to be subjected to special disposal or to recycling, which is an additional burden to the patient. Further, disposing a battery, motor and/or electronics after a single use is a waste of resources and increases the costs of the therapy.

In order to account for a need to handle and dispose the autoinjector parts differently semi-reusable autoinjectors have been developed. Such autoinjectors typically comprise a reusable drive unit as well as a disposable reservoir unit which may be releasably coupled or attached to the drive unit. The drive unit usually includes the drive mechanism and electronics whereas the reservoir unit includes the syringe with the needle and a needle cover. The user can thus discard the reservoir unit when it is empty or after use and can load the drive unit with a new reservoir unit for a new upcoming injection.

WO 2019/191110 discloses a reusable autoinjector with a reservoir unit and a drive unit. A clasp mechanism of the drive unit includes a base latch and a latch lock adapted to retract the reservoir unit. If the clasp mechanism moves the reservoir unit proximally a needle cap mounted on a distal end begins to be separated from the reservoir unit because of mechanical engagement with a distal end of the housing.

WO 2012/145685 discloses a reusable autoinjector with a disposable or single-use cassette and a reusable drive unit. The cassette includes an outer housing, an inner sleeve, a shield remover, a movable cover and a lock cap locking the syringe to the inner sleeve.

WO 2010/046569 discloses a reusable autoinjector with a reservoir unit to be coupled to a drive unit by a bayonet connection. Once a cam of the drive unit is inserted in a slot in the reservoir unit housing a cover sleeve is unlocked and can be moved to unsheathe the needle for an injection. The reservoir unit includes a unit-housing, a shield, a syringe holder and two springs biasing the shield to a covering position.

### DESCRIPTION OF THE INVENTION

It is an objective of the invention to provide for a simple as well as a reliable releasing of a cap, which is mounted to a distal end of an injection device in a shipping state.

This objective is achieved by a safety mechanism and an injection device according to the independent claims. Preferred embodiments are evident from the dependent claims.

The invention relates to safety mechanism comprising a cap and a disposable reservoir unit for an injection device for dispensing a liquid drug through an injection needle. The reservoir unit is configured for releasable attachment to a reusable drive unit of the injection device. The reservoir unit according to the invention comprises, a housing comprising guiding means and defining a longitudinal axis and a needle cover sleeve guided by the guiding means along the longitudinal axis. The needle cover sleeve is movable along the longitudinal axis relative to the housing between a covering position in which the injection needle is covered and a retracted position in which the injection needle exposes and protrudes from the needle cover sleeve. The needle cover sleeve can be further in an initial position before use when the cap is mounted to the distal end of the reservoir unit. The needle cover sleeve is in the initial position when the injection device is in a shipping or initial state and in an unused condition.

The needle cover sleeve comprises a connecting or coupling portion adapted to be releasably coupled to an actuator member of the drive unit. The actuator member can move the needle cover sleeve between the covering position and the retracted position.

The cap of the safety mechanism can be releasably attached to a distal end of the reservoir holder. In the shipping state or in a unused and initial state of the injection device the cap is attached to the reservoir unit such that it covers the injection needle and the cover sleeve is not in its covering position but the needle cover sleeve is then located proximally to the cap and the needle cover sleeve is in contact with the cap or engages the cap. When the needle cover sleeve is moved from its initial position towards its covering position by the actuator the cap is moved form an initial cap position (away from the needle) by the actuator to a release position of the cap such that the cap does not (completely) cover the needle anymore. The needle cover sleeve is adapted to move, preferably shift and in particular shift along the longitudinal axis form an initial cap position into a release position where the cap can be removed. Alternatively, the needle cover sleeve may be adapted to rotate the cap or move the cap with a helical movement from the initial position to the release position. The needle may be partly or entirely covered by the needle cover sleeve when the cap is in its release position after the movement.

According to the invention the injection needle is covered by the cap in a shipping and unused or/and initial state of the injection device. In this shipping state the cap is held onto the reservoir unit in a initial position of the cap. Before an injection the needle cover sleeve is used to remove the cap such that the cap drops off or such that the user can remove the cap from the distal end of the reservoir unit.

With the safety mechanism according to the invention the needle cover sleeve fulfills at least two functions: it is adapted to cover the needle in its covering position and thus prevents unintentional access to the needle. Additionally, the needle cover sleeve is adapted to move the cap away from the injection needle and preferably a sterile barrier is removed from the injection needle. That means the needle cover sleeve is moved by an actuation member (e.g. an arm or plunger driven by a motor or spring member of the drive unit) to release the cap from the reservoir unit. Thus, there is no separate unlock or release mechanism or lock member required as the needle cover sleeve fulfills a cap release function. That allows to reduce the number of parts of the reservoir unit and provides for a simple design. Such a design may help to reduce the quantity of (plastic) material required for the reservoir unit.

Furthermore, the movement of the needle cover sleeve is entirely under control of the drive unit. The actuation member is coupled via coupling portion to the needle cover sleeve and thus the actuation member can move the needle cover sleeve distally or proximally, e.g. between an initial position, the covering position and the retracted position. A biasing or spring member for the cover sleeve within the reservoir unit is not required and may instead be located in the reusable drive unit. That means the reservoir unit is preferably devoid of a cover sleeve spring. Thus, the design of the reservoir unit can be kept simple und reduces the number of required parts.

The injection device may be either a manually actuated or an automatic injection device. Automatic devices or autoinjectors typically include automatic drive means such as, for example, an elastic element (e.g. a pre-tensioned spring) or an electric motor to drive a dispensing member to dispense the liquid drug form the reservoir. Manual injection devices include a manual drive such as a dispensing button that has to be pressed by the user and by a human force to move the dispensing member in dispensing direction.

If the injection device is an automatic device the actuation member moving the needle cover sleeve may be driven, for example, by an electric motor or a biased elastic member. In case the injection device is a manual device the actuation member may be driven manually by a user force.

The reservoir of the injection device may be a prefilled syringe which has a needle or cannula that is permanently attached to a first end of a syringe barrel and that is sealed at the opposing end by a plunger. Alternatively, the reservoir may be a cartridge comprising a needle mounting portion adapted to be releasably connected to a needle assembly prior injection.

The reservoir unit is preferably disposable and is discarded after use. The drive unit is preferably reusable meaning that it can be used for several injection and with different reservoir units. In this case, the injection device may be a semi-reusable injection device and in particular a reusable autoinjector or semi-reusable autoinjector (also named as semi-disposable autoinjector) having the reusable drive unit and the disposable reservoir unit including a syringe or cartridge with the drug.

In these semi-reusable injection device concepts, the reservoir unit is typically not operational without a reusable drive unit of the injection device. To prepare the injection device for an injection the user has to attach or to couple the reservoir unit to the drive unit. The reservoir unit is releasably attachable to the drive unit. That means the two units can be detached and reattached without destroying elements. The reservoir unit is usually attachable to the drive unit by means of a releasable lock such as a snap-fit connection, a thread or a bayonet fitting.

The needle cover sleeve is adapted to cover or envelop the needle of a prefilled syringe or the needle of a cartridge. The movement of the needle cover allows to switch between a safety state in which the needle is covered, and the needle does not protrude from a device housing and an injection state in which the needle is uncovered and exposes or protrudes from the housing and from the needle cover sleeve.

The needle cover sleeve includes a coupling portion for coupling with an actuation member. The coupling portion allows to establish a releasable connection between the needle cover sleeve and the actuation member of the drive unit. By way of example, the coupling portion may be a snap-fit connection, a form fit (form lock) or a thread. The coupling portion allows to transfer a movement of the drive unit actuation member to the needle cover sleeve.

The safety mechanism includes a cap which is mounted on a distal end portion of the reservoir unit in a shipping state where the cap is in its initial position. The cap is adapted to cover the injection needle to prevent unintentional contact with the needle and to avoid injuries. The cap is mounted in the shipping or initial or unused state of the injection device, e.g. before a first use of the reservoir unit. The cap does not only cover laterally the needle (as the needle cover sleeve does) but does also cover a front face and thus completely envelops a distal end of the reservoir unit.

In the shipping state the cap may be releasably attached to the housing in the initial cap position. Instead or additionally, the cap may releasably coupled to the needle cover sleeve or to a further member of the reservoir unit.

Additionally, the cap may be connected to a needle shield, in particular a rigid needle shield (RNS) which provides a sterile barrier to the injection needle. The cap and the needle shield may be removable attached to the reservoir unit. The needle shield may be removable along with the cap.

By preference, upon attachment the reservoir unit is held by the drive unit relative to a drive unit housing such that a movement of the reservoir unit in a dispensing direction is prevented. Furthermore, the reservoir (syringe or cartridge) inside the reservoir unit is preferably fixedly held such that the reservoir cannot move relative to the reservoir unit or relative to the drive unit during the injection process.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection device" or "injector" refers to a device that is removed from the injection site after each medication event or drug delivery process, whereas the term "infusion system" refers to a device with a cannula or needle that remains in the skin of the patient for a prolonged period of time, for example, several hours.

In the claims, the word "comprising" does not exclude other elements or steps, and the indefinite article "a" or "an" does not exclude a plurality. For example, "an arm" does not exclude the fact that there may be two arms that functionally or structurally fulfill the purpose of "an arm". The mere fact that certain elements or steps are recited in distinct claims shall not preclude the existence of further meaningful combinations of these elements or steps.

Preferably, in the shipping state the cap is held onto the reservoir unit by a snap-fit connection and the needle cover sleeve is adapted to release the snap-fit connection exclusively by a movement along the longitudinal axis towards its covering position. That means the needle cover sleeve does not rotate or screw but is exclusively moved linearly in its covering position to release the snap-fit connection.

The snap-fit provides a releasable connection. That means the cap can be released and removed from the reservoir unit by releasing the snap-fit connection in a non-destructive manner. The connection may be released, for example, by a deflection or movement of an elastic member.

The snap-fit provides for an easy and simple connection between the cap and the reservoir unit.

The cap preferably includes a guiding element, for example, a groove, a rail, a rib or a protrusion adapted to engage a counter element such that the cap is guided and can move exclusively along the longitudinal axis and is prevented from being rotated. That ensures a reliable movement of the cap in distal direction when the needle cover sleeve pushes the cap distally.

In a further preferred embodiment in the shipping state the cap is additionally locked by a form fit connection to the reservoir unit such that the cap cannot be pulled off and removed from the reservoir unit by the user. The form fit connection can be released exclusively by a movement of the needle cover sleeve towards its covering position along the longitudinal axis. The needle cover sleeve movement is preferably a linear movement without rotation or screw movement.

That means in this embodiment the needle cover sleeve movement is not only used to remove the cap but previously the movement unlocks a form fit connection (cap lock). The form fit connection or cap lock is an optional and additional precaution to prevent unintentional access and to avoid injuries before use of the injection device for an injection.

The term "locked" means that the cap cannot be removed non-destructively unless the connection is unlocked, e.g. unless the needle cover sleeve is moved along the longitudinal axis. When the needle cover sleeve has been moved from its initial position towards its covering position the cap is unlocked and either falls off without user action or the user may remove the cap from the distal end of the reservoir unit.

Preferably, the snap-fit connection comprises a flexible arm extending along the longitudinal axis and a recess adapted to accommodate the arm in a connected state. The flexible arm is adapted to be deflected, preferably in a plane parallel to the longitudinal axis, to release the snap-fit connection and to release the cap from the reservoir unit.

The arm extends preferably along the longitudinal axis and is deflectable in a plane parallel to the longitudinal axis. That allows for a space-saving design. Preferably, the deflectable arm is deflectable in a plane tangent to a circumferential surface of the needle cover sleeve (e. g. tangent to a cylindrical shape of the needle cover sleeve). That means the arm does not deflect exclusively radially inwards towards a center of the reservoir unit or in a plane including the device axis but instead it can deflect in a plane parallel to the longitudinal axis and tangent to the cylindrical shape of the needle cover sleeve. In a specific embodiment, the deflectable arm may deflect along a circumferential direction such that only a movement component is in the plane tangent to the cover sleeve.

The arm may be integrally formed in the cap and the recess may be formed by the housing or alternatively, the arm may be provided by the housing and the recess may be provided by the cap.

The arm and the recess provide a reliable and an easy connection between the cap and the housing.

In order to facilitate the release of the connection between the cap and the housing the arm preferably includes a sloped surface and the recess includes a counter sloped surface. When the sloped surface and the counter sloped surface slide along each other the arm is preferably deflected and the connection is released.

In a further preferred embodiment, the cap includes the deflectable arm and the housing includes the recess and the needle cover sleeve includes a stop member which provides the form fit connection and thus the stop member provides the cap lock. The stop member is adapted to prevent a deflection of the arm when the cover sleeve is in an initial position relative to the cap in the shipping state. That means in this position the stop member is positioned in a stop position which is in a travel path of the deflectable arm such that the arm is hindered when it starts to deflect. The snap-fit connection can thus not be released when the stop member is in this stop position.

When the cover sleeve is moved relative to the cap (and relative to the housing) towards the covering position the stop member is moved away from the arm and out of its stop position such that the arm is no longer prevented from being deflected. That means the stop member is then no longer in the travel path of the deflectable arm. The form fit connection and thus the cap lock is then released although the cap is still held on the housing.

At the beginning of this relative movement between the needle cover sleeve and the cap, the latter is held in place and held on the housing by the snap-fit connection. When the needle cover sleeve is further moved distally the snap-fit connection is released because the needle cover sleeve has pushed the cap in distal direction.

The stop member may be, for example, a rib, a protrusion, a rail or a wall. Preferably, the stop member has an elongated form and extends along the longitudinal axis. The stop member can then reliably prevent the arm from being deflected when the needle cover sleeve is in its shipping or initial position (and the stop member is thus in its stop position).

Preferably, the cap includes a first and a second arm both extending along the longitudinal axis and the stop member is arranged in-between the two arms in the shipping or initial position of the needle cover sleeve.

In a preferred embodiment, if the cap is attached to the reservoir unit a first side of the stop member prevents the first arm from being deflected and correspondingly a second opposite side of the stop member prevents the second arm from being deflected. If the needle cover sleeve is moved towards the covering position the first and second arm can deflect towards each other and thus the cap is released and can be moved distally and subsequently, it can be removed from the reservoir unit. The embodiment including two arms may increase the reliability in view of locking and unlocking of the cap.

The needle cover sleeve movement towards the covering position is preferably a linear and non-rotating movement exclusive along the longitudinal axis. That allows for a space-saving design as no rotational guide is required.

In the initial position, the needle cover sleeve is preferably positioned such that a distal end of needle cover sleeve directly contacts a contact surface of the cap such that the longitudinal movement of the needle cover sleeve causes the cap to move distally. Thus, by moving the needle cover sleeve distally in towards its covering position the needle remains reliably covered such that unintentional access to the needle is prevented during the entire cap releasing and cap removing process. The movement along the longitudinal axis provides an easy and quick unlock and covering of the needle.

That means the movement of the needle cover sleeve towards its covering position directly moves the cap distally to unshield or/and preferably to remove a sterile barrier of the injection needle. In a preferred embodiment the cover sleeve movement additionally unlocks and release the cap as described above.

Preferably, the needle cover sleeve or the housing includes a snap element adapted to non-releasably snap into a recess of the other of the needle cover sleeve and the housing when the needle cover sleeve is in a distal end position such that the needle cover sleeve is locked in the distal end position and cannot anymore be moved out of the end position without being destructed.

The distal end position may be distally to the covering position or it may be identical to the covering position. A switch, a clutch or a cam control may be implemented ensuring that the needle cover sleeve does not lock too early to the housing before the injection but only after the injection, e.g. when the needle cover sleeve has already been in the retracted position.

The non-releasably snap fit between the needle cover sleeve and the housing ensures that the needle remains covered after injection and thus unintentional access to the needle and injuries are prevented.

Preferably, the snap element or the recess includes a sloped surface adapted to deflect the snap element and subsequently snap or engage to the recess when the needle cover sleeve is moved into the distal end position.

The sloped surface thus provides a reliable engagement of the snap element with the recess and thus a reliable locking of the needle cover sleeve after the injection.

Preferably, the safety mechanism further includes the reservoir with an injection needle and a needle shield mounted onto the injection needle and shielding the injection needle in the shipping state. Furthermore, in a preferred embodiment the needle cover sleeve includes a radially inwards protruding arm. In the shipping state the arm is positioned between a proximal end of a needle shield and a distal shoulder of the reservoir, wherein during the needle cover sleeve movement towards its covering position the arm abuts the needle shield and moves the needle shield distally thereby removing a sterile barrier.

During a movement of the needle cover sleeve from the initial position towards the covering position the arm causes the needle shield to move distally along with the cap. When the cap and the needle shield are moved distally the sterile barrier of the injection needle is removed.

The needle shield may be a rigid needle shield (RNS) shielding and covering the injection needle in the shipping state or unused state and provides the sterile barrier.

The inwards protruding arm is preferably flexible and/or flexibly connected to the rest of the needle cover sleeve such that the element is deflectable in order to pass the needle shield or the reservoir during an assembly when the reservoir is mounted inside the reservoir unit.

In a preferred embodiment the cap is fixedly connected to the needle shield, for example by clamping elements (force fit) or by holding elements engaging counter elements on the needle shield (form fit). Thus, if either the needle shield or the cap or both are moved distally by the needle cover sleeve the other of the needle shield or cap is moved distally too.

The housing includes preferably a circumferential recess adapted to accommodate a proximal flange of the reservoir such that the reservoir is non-movably and fixedly held by the housing. That means the reservoir cannot move relative to the housing. The recess allows to reliable held the reservoir in place. Alternatively, the reservoir may be clamped or held by a contact surface abutting a distal shoulder of the reservoir.

In a preferred embodiment the reservoir unit comprises in total not more than two separate injection-molded plastic parts apart from the reservoir. That means in this embodiment the reservoir unit comprises or consists of in total at maximum two separate plastic parts, preferably the two parts are separately produced. The two parts are preferably the housing and the needle cover sleeve.

The reservoir itself is not counted. Reservoir elements not counted may include the injection needle separately connected to a glass barrel, a piston movably arranged inside the barrel and a needle shield (e.g. RNS) directly mounted onto the metallic injection needle in the shipping state.

The manufacturing, the assembly, the handling and storage of the reservoir unit is simplified compared to reservoir units with more parts. A reduced number of parts can thus help to reduce the overall production costs and reduce the complexity of the reservoir unit. Besides that, the number of discarded parts and thus the waste can be reduced which is advantageous from an environmental perspective.

Preferably, the housing and the needle cover are made of injection-molded plastics and the reservoir unit apart from the reservoir is devoid of metallic parts. The needle cover sleeve and the housing are preferably thermoplastic plastic parts. That means the reservoir unit is devoid of any metal parts such as springs, clamps or the like. This facilitates the production and contributes to a cost-efficient design.

The housing is preferably sleeve-shaped and the needle cover sleeve is coaxially inside arranged to the housing. The housing is thus the outermost part of the reservoir unit. As described above the housing includes guiding means such as, for example, a rail, a groove or a spline and the needle cover sleeve includes a counter element adapted to engage the guiding means such that the needle cover sleeve is exclusively movable along the longitudinal axis.

The sleeve-shaped housing may include a lateral opening extending along the longitudinal axis. The coupling portion of the needle cover sleeve is design and arranged to travel long the longitudinal axis and inside the opening such that the coupling portion is accessible through the lateral opening during a movement of the needle cover sleeve between the initial position, the covering position and the retracted position.

That means the actuation member of the drive unit may remain connected to the cover sleeve during movement and is not impeded or hindered by the housing. The lateral opening has preferably an elongated shape and extends axially. The lateral opening itself may provide the guiding means to guide the needle cover sleeve exclusive along the longitudinal axis.

The invention relates further to an injection device comprising the above-described safety mechanism. The reservoir unit is preferably disposable and the drive unit reusable. The drive unit may include a plunger rod and a drive for moving the plunger rod in a dispensing direction to dispense the drug from the reservoir.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts a perspective view of a reservoir unit according to the invention;
- Fig. 2: depicts an exploded view of the reservoir unit of figure 1;
- Fig. 3a: depicts a sectional and perspective view of the reservoir unit in a shipping state;
- Fig. 3b: depicts a top view of the reservoir unit in the shipping state;
- Fig. 4a: depicts the reservoir unit in a state when the cap is unlocked;
- Fig. 4b: depicts the top view of the reservoir unit when the cap is unlocked;
- Fig. 5: depicts the reservoir unit with the needle cover sleeve in a retracted position;
- Fig. 6a: depicts a sectional view of the reservoir unit after an injection when the needle cover sleeve is in a locked position;
- Fig. 6b: depicts a perspective view of the reservoir unit when the needle cover sleeve is in the locked position and
- Fig. 7: depicts a schematical view of the injection device with the reservoir unit attached to the drive unit.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

In the present description the term "distal" refers to the side where the injection needle is located. This is on the left-hand side in the figures. The term "proximal" refers to the opposite side or rear end of the device and is on the right-hand side in the figures.

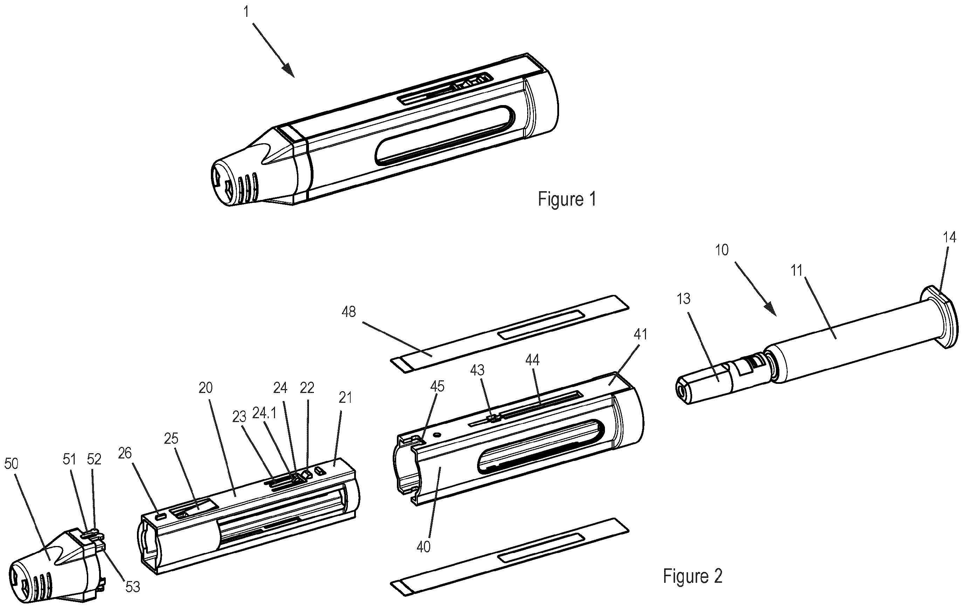

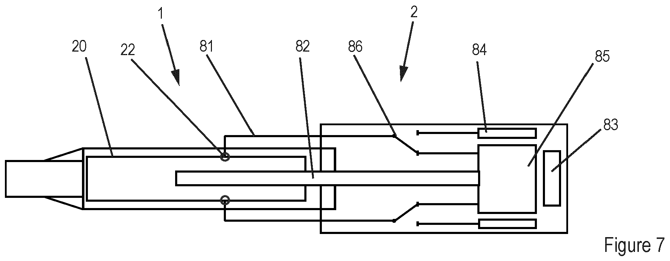

Figure 1 shows a perspective view of a reservoir unit 1 with a safety mechanism according to the invention for a semi-reusable autoinjector. The autoinjector comprises the disposable reservoir unit 1 and a reusable drive unit 2 as schematically shown in figure 7. The reservoir unit 1 is releasably attachable to the drive unit 2. The drive unit 2 includes an opening or cavity adapted to accommodate a proximal portion of the reservoir unit 1 for coupling the reservoir unit to the drive unit 2.

As it can be seen figure 1 and 2 the reservoir unit 1 has an elongated housing defining a longitudinal axis. Figure 2 depicts an exploded view of the reservoir unit. As shown in figure 2 the reservoir unit accommodates a reservoir in form of a prefilled syringe 10 containing a liquid drug.

In the following the structural features of the reservoir unit 1 will be descripted in detail. Subsequently, the function of the semi-reusable autoinjector 1 will be explained.

As shown in figure 1 and 2 the reservoir unit 1 has two oppositely arranged flattened sides 41. On a distal end a cap 50 is releasably mounted in a shipping state. A proximal end of the reservoir unit includes a circumferential holding structure (not shown) to releasably attach the reservoir unit 2 to the drive unit 3.

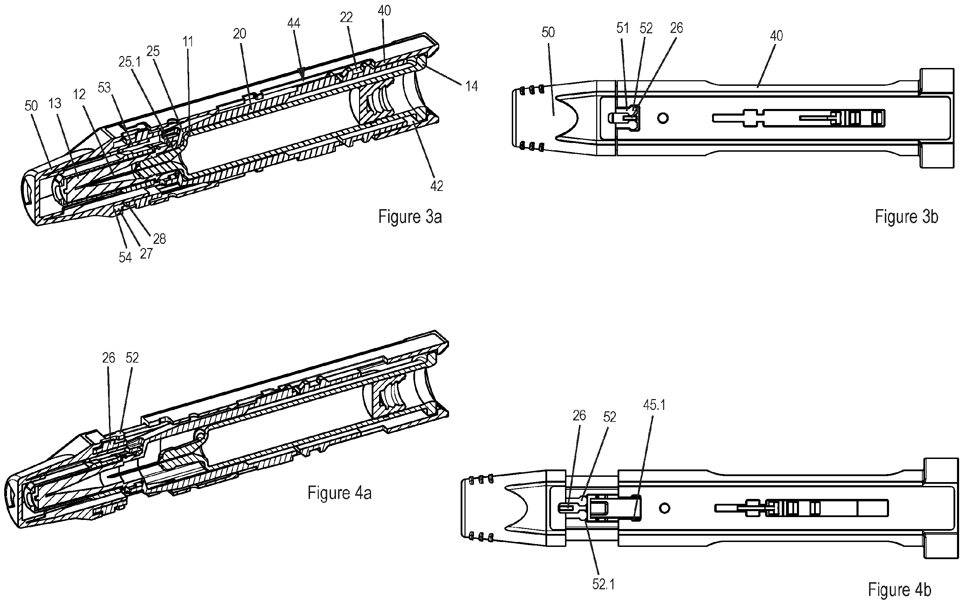

As best shown in figure 2 the reservoir unit 1 includes two injection-molded plastics parts, namely an outer housing 40 and a coaxially inside arranged needle cover sleeve 20 which is movable relative to the housing 40 along the longitudinal axis. The reservoir unit 1 further includes two labels 48 attached to the flattened sides 41 Alternatively, it is a one-piece the label wrapped around the housing. Inside the reservoir unit the prefilled syringe (PFS) 10 comprising the liquid drug or medication is arranged. The syringe may have a capacity, for example, of 1 ml or 2.25 ml. The syringe includes a barrel 11 made of glass (or alternatively made of plastics) and an injection needle 12 (see figure 3a) permanently fixed to the barrel 11 at a distal end. A rigid needle shield (RNS) 13 is mounted on the injection needle in a shipping state.

The prefilled syringe 10 is axially fixed inside the housing 40. If the syringe includes a cut-flange the syringe is additionally rotationally fixed relative to the housing. For that purpose, the sleeve-shaped housing includes in a proximal end portion on its inside a circumferential groove 42 (figure 3a) accommodating a proximal flange 14 of the syringe 10. The syringe 10 is thus fixedly connected to the housing 40 and cannot move relative thereto.

As best shown in figure 2 the needle cover sleeve 20 has also two oppositely flattened sides 21 that are guided in an inside of two flattened sides 41 of the housing 40. The housing thus provides a linear guide such that the needle cover sleeve 20 can be shifted exclusively along the longitudinal axis relative to the housing 40. On each flattened side 21 the needle cover sleeve 20 includes in a proximal portion a coupling portion in form of two protrusion forming a coupling clamp 22 which is adapted to be releasably coupled with an actuation arm 81 (figure 7) of the drive unit 2 as will be described below.

Distally to the coupling clamp 22 the needle cover sleeve 20 include on each side a snap element 23 with a nose 24 on its free end. The latter features a sloped contact surface 24.1 to facilitate deflection of the snap element 23. The snap element is adapted to snap into a recess 43 in the housing after injection when the needle cover sleeve 20 is in a distal end position.

In a distal portion, the needle cover sleeve comprises on its flattened sides 21 two inwardly protruding flexible arms 25. Each arm 25 includes an inwardly extending cam 25.1 on its free end (see figure 3a). During assembly the prefilled syringe 10 with the RNS 13 mounted on the injection needle 12 is inserted from a proximal side into the needle cover sleeve 20. During insertion in distal direction the RNS 13 abuts the cams 25.1 of the flexible arms 25 and if the syringe 10 is further moved distally the flexible arms 25 radially deflect to pass the RNS 13. Subsequently, the arms 25 move back into their initial non-deflected state such that the cams 25.1 are located between a proximal end of the RNS 13 and a distal shoulder of the syringe barrel 11. A distal side of the cams 25.1 thereby contact or abut a proximal end surface of the RNS 13. This assembled state is shown in figure 3a.

As seen in figure 2 the needle cover sleeve further comprises a longitudinal extending blocking rib 26 on its flattened sides 21. The blocking rib 26 is located distally to the flexible arms 25. As described below in detail the rib 26 is designed to lock cap arms in a holding position.

As best shown in figure 2 the sleeve-shaped housing 40 comprises on each flattened side 41 a longitudinal opening 44 through which the coupling protrusion 22 of the needle cover sleeve is accessible from outside. Distally to the opening the housing includes the recess 43 in which the snap element 23 of the needle cover sleeve 20 can be engaged. At a distal, end the housing 40 features a T-shaped groove 45 or opening adapted to accommodate the cap arms 51.

On both flattened sides labels 48 are attached to the housing. One label 45 provides a QR code or it may include a RFID code integrated in the label. The code can include information about the reservoir unit for identification and may include further information about the drug inside the syringe, the volume, expire date and dispensing parameter such as injection speed and holding time The code may have further country-specific information such as a user language to adjust a user interface language or it may refer to a product IFU.

As mentioned above in a shipping state, a cap 50 is releasably mounted on a distal end of the reservoir unit 1 in an initial position of the cap. The cap 50 includes two oppositely arranged pairs of proximally extending and parallel flexible cap arms 51. The arms 51 include a knob-shaped end portion 52 on their free ends. The end portions 52 include a sloped surface designed to facilitate deflection of the arms when the cap 50 is moved distally to release from the housing. The knob-shaped portions 52 are located in the T-shaped groove 45 of the housing if the cap 50 is mounted. As best shown in figures 3a and 4a the cap 50 accommodates the RNS 13 on its inside. The RNS 13 is fixedly connected to the cap 50 by clamping arms (not shown) such that the RNS 13 is removed from the injection needle when the cap 50 is removed from the reservoir unit 1. Alternatively, the cap may include an inner sleeve or holder connected to the RNS. In this case the inner holder is made of an elastomer and is shrunken onto the RNS during assembly of the reservoir unit. As an outer part of the cap made of plastics the holder and the outer cap from a two-component injection molded part. The cap with the holder is descripted in the patent application EP23165348.6 (Ypsomed) in detail which is incorporated herein by reference.

The cap 50 additionally includes two oppositely arranged guiding ribs 53 (figure 2 and 3a) on an inside of the cap. The ribs 53 are guided within corresponding axial grooves in the needle cover sleeve 20 on the inside of the flattened portions 21. The cap 50 is thus axially guided and prevented from being rotated relative to the needle cover sleeve 20.

As mentioned above the reservoir unit 1 is releasably attachable to the drive unit 2. For that the housing 40 may include coupling elements such as protrusions or grooves for a bayonet connection between the reservoir unit 1 and the drive unit 2.

Figure 7 depicts a schematic view of the autoinjector 3 with the disposable reservoir unit 1 and the reusable drive unit 2. The drive unit 3 comprises a plunger rod 82, a drive 85 for moving the plunger rod in distal direction for dispensing the drug, a control unit 83, two oppositely arranged actuation arms 81 and a cover sleeve spring 84 that can be coupled to the needle cover sleeve via actuation arms. The actuation arms 81 are driven by the drive and releasably coupled to the needle cover sleeve such that the arms 81 moves along the longitudinal axis distally and proximally. The arms 81 includes on its free end a counter coupling element, such as a cam, adapted to releasably engage the coupling clamp 22 of the needle cover sleeve 20 upon insertion of the reservoir unit 1 into the drive unit and coupling the reservoir unit to the drive unit.

In a first embodiment the drive 85 is an electric motor coupled to the plunger rod 82. The control unit 83 controls the motor to drive the plunger rod according to a dose to be dispensed. The motor can further drive the actuation arms 81 to hold and move the needle cover sleeve 20 along the longitudinal axis. Namely, the motor drives the actuation arms 81 to move the needle cover sleeve 20 from an initial or shipping position into its covering position to remove the cap 50 as described below.

In an alternative embodiment the drive 85 may be a mechanical spring or it may be a button that has to be pressed manually by a user. In this case the plunger rod is moved by a user force. In this embodiment the user moves the actuation arms 81 distally.

The actuation arms 81 are coupled to the drive 85 via clutch 86 or coupler which ensures that the drive 85 is coupled to the actuation arms 81 for moving the needle cover sleeve 20 distally in its covering position. The clutch 86 can then decouple the actuation arms 81 from the drive 85 such the needle cover sleeve 20 can be moved from its covering position into its retracted position by the user (push on skin). In the decoupled state the needle cover sleeve 20 is coupled and biased by the cover sleeve spring 84 biasing the needle cover sleeve 20 in distal direction. The clutch 86 can be implemented by a cam control or mechanical switch adapted to selectively couple and decouple the drive 86 to actuation arms 81 and to selectively couple the actuation arms 81 to the cover sleeve spring 84.

In the following the function of the reservoir unit is descripted in detail.

Figure 3a depicts a sectional view of the reservoir unit without drive unit where the cut for the section runs along the longitudinal axis. Figure 3a shows a shipping or unused state of the reservoir unit 1. The cap 50 is attached to the distal end of the reservoir unit and the RNS 13 is mounted onto the injection needle 12.

Figure 3b depicts a top view of the reservoir unit 1 in the same state as in figure 3a. As best shown in figure 3b the cap 50 is locked to the reservoir unit and cannot be pulled off by the user. The lock is provided by the blocking rib 26 of the needle cover sleeve arranged in between the two knob-shaped end portions of the longitudinally extending cap arms 51 and thereby preventing a deflection of the cap arms 51.

When the reservoir unit 1 is attached and coupled to the drive unit the actuation arms 81 of the drive unit 2 reach through the openings 44 to the clamps 22 on both sides and the arms 81 are thus coupled to the needle cover sleeve 20 such that an axial movement of the actuation arms is transferred to the needle cover sleeve. The actuation arms and the drive unit are not shown in figures 3a to figure 6b.

A tag reader of the drive unit reads the code on the label 48 and verifies the read information with a database. After successful verification the control unit enables the drive 85 to move the actuation arms 81 in distal direction. That causes the needle cover sleeve 20 to move distally. As best shown in figure 3a in the shipping state a gap 27 of 2 to 4 mm is present between a distal end 28 of the needle cover sleeve 20 and a proximal contact surface 54 of the cap 50. That means when the needle cover sleeve 20 starts to move distally it moves relative to the cap for the length of the gap 27. That means the blocking rib 26 is moved out of engagement and away from the knob portions 52 of the arms 51 meaning that the arms 51 are no longer prevented from being deflected inwardly towards each other. The cap lock is then released. This state is shown in figure 4a and 4b. As it can be seen in these figures the blocking rib 26 is has been moved distally in comparison with figures 3a and 3b and the rib 26 is no longer in between the end portions 52 of two cap arms 51.

When the actuation arms 81 move further in distal direction the distal end 28 of the needle cover sleeve 20 gets into contact with the cap proximal contact surface 54 and then pushes the cap 50 distally out of its initial position towards a cap release position. As the end portions 52 of the cap arms 51 are then deflected radially inwards by their sloped surfaces and by the narrow portion of the T-shaped groove 45 the arms 51 subsequently get out of the groove 45. As the RNS 13 is fixedly connected to the cap 50 the RNS 13 is moved distally too. The sterile barrier is therefore removed from the injection needle. The actuation arms 81 driven by the drive 85 further move the needle cover sleeve 20 distally until the position as shown in figure 4a and 4b is reached. The cap is then in its release position. In the state shown in figure 4a and 4b the cap lock is released and the cap is ready to be pulled off by the user in distal direction. The actuation arms 81 are in a distal end position and are then decoupled from the drive 86 but coupled to the cover sleeve spring 84 by the clutch 86. The cover sleeve spring 84 biases the needle cover sleeve 20 towards its covering position.

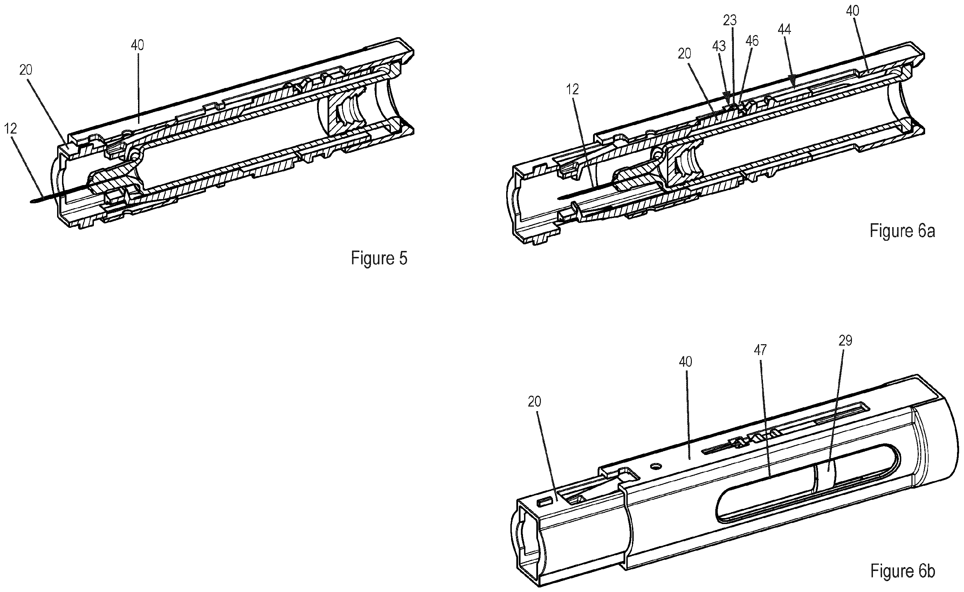

The autoinjector 3 is then ready for an injection. To start an injection the user presses the distal end of the needle cover sleeve 20 onto the injection site (push on skin). That means the needle cover sleeve 20 and also the actuation arms 81 are pushed proximally against the force of the cover sleeve spring 84 until the actuation arms 81 reaches a proximal end stop provided by the drive unit. The reservoir unit 1 in this state is shown in figure 5. As shown in figure 5 the needle 12 protrudes from the needle cover sleeve 20 which is in its retracted position. Reaching the proximal end stop generates start signal for the drive to move the plunger rod in dispensing direction to dispense the drug from the syringe.

When the injection is completed, the user removes the autoinjector 3 from the injection site. The needle cover sleeve 20 is then moved distally again from its retracted position into a distal end position by the force of the biased cover sleeve spring 84. In contrast to the initial distal movement described above the actuation arms 81 are decoupled from the drive 85 and the drive does not stop the needle cover sleeve 20 in a distal position (covering position). Instead, the force of the cover sleeve spring moves the needle cover sleeve 20 into the distal end position which is distally to the covering position and in which the injection needle 12 is completely covered by the needle cover sleeve 20. Shortly before the distal end position is reached a rib 46 (figure 6a) in the housing 40 located between the longitudinal opening 44 and the recess 43 causes the snap element 23 to deflect by its sloped surface such that the snap element 23 snaps into the recess 43 in the housing. This state is depicted in figure 6a which shows a sectional view of the reservoir unit with the needle cover sleeve 20 in its distal end position. That means the needle cover sleeve 20 is then locked to the housing 40 and cannot be moved proximally again. A needle cover sleeve lock is thus active.

Figure 6b depicts a perspective view and sectional view of the reservoir unit where the needle cover sleeve 20 is locked in the distal end position. As shown in figure 6b a connecting strip or indicator 29 of the needle cover sleeve 20 appears in a lateral viewing window 47 in the housing 40 indicating the user that the needle cover sleeve 20 is in its distal end position and that the needle cover sleeve is locked.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

**LIST OF DESIGNATIONS**

| | | | |
|---|---|---|---|
| 1 | reservoir unit | 50 | cap |
| 2 | drive unit | 51 | arms |
| 3 | autoinjector | 52 52.1 | end portion sloped surface |
| 10 | syringe | 53 | guiding rib |
| 11 | barrel | 54 | proximal end surface |
| 12 | injection needle | | |
| 13 | rigid needle sleeve RNS | 81 | actuation arm |
| 14 | flange | 82 83 | plunger rod control unit |
| 20 | cover sleeve | 84 | cover sleeve spring |
| 21 | flattened sides | 85 | drive |
| 22 | coupling clamp | 86 | clutch |
| 23 | snap element | 87 | coupling portion |
| 24 | nose | | |
| 24.1 | sloped surface | | |
| 25 | flexible arms | | |
| 25.1 | cam | | |
| 26 | blocking rib | | |
| 27 | gap | | |
| 28 | distal end | | |
| 29 | indicator | | |
| 40 | housing | | |
| 41 | flattened side | | |
| 42 | groove | | |
| 43 | recess snapper | | |
| 44 | longitudinal opening | | |
| 45 | T-shaped groove | | |
| 45.1 | sloped surface | | |
| 46 | housing rib | | |
| 47 | viewing window | | |
| 48 | label | | |

## Claims

1. A safety mechanism comprising a cap (50) and a disposable reservoir unit (1) for an injection device (3) for dispensing a liquid drug through an injection needle (12), the reservoir unit (1) being configured for releasable attachment to a reusable drive (2) unit of the injection device (3), the reservoir unit (1) comprising:
a housing (40) comprising guiding means and defining a longitudinal axis;
a needle cover sleeve (20) guided by the guiding means and movable along the longitudinal axis relative to the housing (40) between a covering position in which the injection needle (12) is covered and a retracted position in which the injection needle (12) protrudes from the needle cover sleeve (20) and
wherein the needle cover sleeve (20) comprises a coupling portion (22) adapted to be releasably coupled to an actuation member (81) of the drive unit (2) to move the needle
cover sleeve (20) between the covering position and the retracted position;
**characterized in that** in a shipping state of the reservoir unit the cap (50) is attached to a distal end of the reservoir unit (1) in an initial position such that the cap (50) covers the injection needle (12) and the needle cover sleeve (20) is proximally to the cap and not in its covering position, wherein reservoir unit is adapted such that when the needle cover sleeve (20) is moved towards its covering position by the actuator member (81) of the drive unit (2) the cap (50) is moved from its initial position to a release position by the needle cover sleeve (20).

2. Safety mechanism according to claim 1, wherein in the shipping state the cap (50) is held onto the reservoir unit (1) by a snap-fit connection and the needle cover sleeve (20) is adapted to release the snap-fit connection exclusively by the movement towards its covering position along the longitudinal axis.

3. Safety mechanism according to claim 1 or 2, wherein in the shipping state the cap (50) is locked by a form fit connection to the reservoir unit (1) such that the cap (50) cannot be pulled off from the reservoir unit (1) and wherein the form fit connection can be released exclusively by the needle cover sleeve movement towards the covering position along the longitudinal axis.

4. Safety mechanism according to claim 2 or 3, wherein the snap-fit connection comprises a flexible arm (51) and a recess (45) adapted to accommodate the arm (51) in a connected state and wherein the arm (51) is adapted to be deflected to release the connection.

5. Safety mechanism according to claim 4, wherein the arm (51) includes a sloped surface (52.1) and the recess includes a counter sloped surface (45.1), wherein when the sloped surface (52.1) and the counter sloped surface (45.1) slide along each other the arm (51) is deflected.

6. Safety mechanism according to claim 3 and claim 4 to 5, wherein the cap (50) includes the deflectable arm (51) and the housing (40) includes the recess (45) and the needle cover sleeve includes a stop member (26) providing the form fit connection and wherein the stop member (26) is adapted to prevent a deflection of the arm (51) when the needle cover sleeve (20) is in an initial position relative to the cap (50), wherein when the needle cover sleeve (20) is moved towards its covering position the stop member (26) is moved away from the arm (51) such that the arm (51) is no longer prevented from being deflected.

7. Safety mechanism according to any of claims 1 to 6, wherein the needle cover sleeve (20) movement towards the covering position is a linear and non-rotating movement exclusive along the longitudinal axis.

8. Safety mechanism according to any of claims 1 to 7, wherein the needle cover sleeve (20) or the housing (40) includes a snap element (23) adapted to non-releasably snap into a recess (43) of the other of the needle cover sleeve (20) and the housing (40) when the needle cover sleeve (20) is in a distal end position such that the needle cover sleeve (20) is locked in the distal end position and cannot any more be moved out of the end position.

9. Safety mechanism according to claim 8, wherein the snap element (23) or the recess (43) includes a sloped surface adapted to deflect the snap element (23) to snap the snap element into the recess (43).

10. Safety mechanism according to any of claims 1 to 9, wherein the needle cover sleeve (20) includes a radially inwards protruding arm (25), wherein in the shipping state the arm (25) is positioned between a proximal end of a needle shield (13) shielding the injection needle (12) in the shipping state and a distal shoulder of the reservoir (10), wherein during the needle cover sleeve movement towards its covering position the arm (25) abuts the needle shield (13) and moves the needle shield (3) distally thereby removing a sterile barrier.

11. Safety mechanism according to any of claims 1 to 10, wherein the housing (40) includes a circumferential groove (42) adapted to accommodate a proximal flange (14) of the reservoir (10) such that the reservoir is non-movably held by the housing (40).

12. Safety mechanism according to claim 1 to 11, wherein apart from the reservoir (10) the reservoir unit (1) comprises in total not more than two separate injection-molded plastic parts.

13. Safety mechanism according to any of claims 1 to 12, wherein the housing (40) is sleeve-shaped and the needle cover sleeve (20) is arranged coaxially inside the housing.

14. Safety mechanism according to claim 13, wherein the sleeve-shaped housing (40) includes a lateral opening (44) extending along the longitudinal axis, wherein the coupling portion (22) is design and arranged to travel long the longitudinal axis inside the lateral opening (44) and accessible therethrough during a movement of the needle cover sleeve (20) between the covering position and the retracted position.

15. Injection device comprising the safety mechanism according to any of claims 1 to 14 and the reusable drive (2) unit including a plunger rod (82) and a drive (85) for moving the plunger rod (82) in a dispensing direction to dispense the drug from the reservoir (10), wherein the reservoir unit (1) is releasably attachable to the drive unit (2).
